# EUROPEAN PATENT APPLICATION

(11) **EP 4 029 482 A1**
(43) Date of publication of application: **20.07.2022**
(21) Application number: 21217865.1
(22) Date of filing: 27.12.2021
(51) Int. Cl.: A61F 13/00

(54) **MEDICAL ARTICLE**

(30) Priority: 29.12.2020 IT 202000007127 U
(71) Applicant: B-Com S.r.l., 25010 San Zeno Naviglio (BS) (IT)
(72) Inventor: BRAGA, Carlo, 25010 BORGOSATOLLO BS (IT); FORMA, Ornella, 21026 GAVIRATE VA (IT)
(74) Representative: Modiano, Micaela Nadia

(57) **Abstract**

A medical article comprising a base body made of non-woven fabric having a percentage at least equal to 50% of bamboo viscose, the base body being impregnated with a sodium chloride solution.

## Description

The present invention relates to a medical article.

Sterile gauzes are known and widely used, for example to treat wounds or abrasions.

These gauzes are typically made of cotton.

In some case, in order to sterilize the wound or abrasion, the gauzes are impregnated, before being placed in contact with the wound, with a saline solution so as to disinfect it.

Known solutions, though widely used, are however not free from drawbacks.

In particular, a certain inconvenience is noted in impregnating the gauze with saline solution on the spot.

Furthermore, cotton does not release such saline solution in a controlled manner.

The aim of the present invention is to provide a medical article that is capable of improving the background art in one or more of the aspects indicated above.

Within this aim, an object of the invention is to provide a medical article that is easy and straightforward to use.

A further object of the invention is to provide a medical article that is highly reliable, relatively easy to provide and at competitive costs.

This aim, as well as these and other objects which will become better apparent hereinafter, are achieved by a medical article according to claim 1, optionally provided with one or more of the characteristics of the dependent claims.

Further characteristics and advantages of the invention will become better apparent from the description of a preferred but not exclusive embodiment of the medical article according to the invention.

A medical article according to the invention comprises a base body made of non-woven fabric which has a percentage of bamboo viscose at least equal to 50%.

The base body is impregnated with a sodium chloride solution.

In greater detail, the sodium chloride solution has a percentage of sodium chloride comprised between 18% and 25%.

Preferably, the sodium chloride solution has a percentage of sodium chloride comprised between 20% and 22%.

Conveniently, the base body made of non-woven fabric has a percentage at least equal to 75% of bamboo viscose.

According to a preferred embodiment, the base body made of non-woven fabric is made of bamboo viscose.

The medical article according to the invention is a dressing mainly indicated to keep under control the microenvironment of a lesion, in particular to manage critically colonized/infected ulcers and/or surgical wounds.

The medical article according to the invention is also suitable to be used in negative-pressure wound therapy (NPWT).

In contact with the exudate, the medical article, by osmotic effect, facilitates the debridement of slough and fibrin and at the same time, by virtue of the high salt concentration, creates a microenvironment that is hostile to bacteria.

Furthermore, due to the high concentration of sodium chloride, the reverse osmosis that is created allows a reduction of perilesional skin edema.

It is recommended not to use the medical article according to the invention on poorly exuding lesions or on dry necrotic tissue.

The use of the medical article according to the invention is as follows.

After normal sanitization operations, it is necessary to clean the bottom of the lesion and the perilesional skin with a detergent solution, leaving a gradient of humidity with said solution.

In this regard, it is recommended to perform a compress with a detergent solution or antiseptic in relation to the phase in which the lesion is.

The medical article must be applied to the bottom of the lesion up to the margin, without covering the perilesional skin.

In the presence of cavitary lesions, use the medical article as a plug.

Depending on the quantity of exudate, it is possible to use secondary dressings, both with the traditional method (gauze and plaster) and with the advanced method (e.g., polyurethane foam, alginates) with an absorbent action.

In practice it has been found that the medical article according to the invention achieves the intended aim and objects.

The medical article according to the invention is a sterile dressing that allows, by means of an osmotic action, the removal of the infectious agents, controlling local infection and the bacterial load.

Moreover, the medical article also helps to control highly exuding wounds and facilitates debridement on the bottom of the lesion.

The invention thus conceived is susceptible of numerous modifications and variations, all of which are within the scope of the accompanying claims; all the details may furthermore be replaced with other technically equivalent elements.

In practice, the materials used, so long as they are compatible with the specific use, as well as the contingent shapes and dimensions, may be any according to the requirements and the state of the art.

The disclosures in Italian Utility Model Application No. 202020000007127, from which this application claims priority, are incorporated herein by reference.

## Claims

1. A medical article, **characterized in that** it comprises a base body made of non-woven fabric which has a percentage at least equal to 50% of bamboo viscose, said base body being impregnated with a sodium chloride solution.

2. The medical article according to claim 1, **characterized in that** said sodium chloride solution has a percentage of sodium chloride comprised between 18% and 25%.

3. The medical article according to one or more of the preceding claims, **characterized in that** said sodium chloride solution has a percentage of sodium chloride comprised between 20% and 22%.

4. The medical article according to one or more of the preceding claims, **characterized in that** said base body made of non-woven fabric has a percentage at least equal to 75% of bamboo viscose.

5. The medical article according to one or more of the preceding claims, **characterized in that** said base body made of non-woven fabric is made of bamboo viscose.
